# FASCICULE DE BREVET EUROPEEN

(11) **EP 1 066 030 B1**
(45) Date de publication et mention de la délivrance du brevet: **14.02.2007**
(21) Numéro de dépôt: 99909078.0
(22) Date de dépôt: 23.03.1999
(51) Int. Cl.: A61K 9/06, A61K 9/70, A61K 31/57

(54) **COMPOSITION HORMONALE TOPIQUE A EFFET SYSTEMIQUE**
TOPISCHE HORMONHALTIGE ZUSAMMENSETZUNG MIT SYSTEMISCHER WIRKUNG
TOPICAL HORMONAL COMPOSITION WITH SYSTEMIC EFFECT

(30) Priorité: 23.03.1998 FR 9803533
(43) Date de publication de la demande: 10.01.2001
(73) Titulaire: LABORATOIRE THERAMEX, 98000 Monaco (MC)
(72) Inventeur: LANQUETIN, Michel, 06340 La Trinité (FR); PARIS, Jacques, Le Clos de Cimiez, 06100 Nice (FR); THOMAS, Jean-Louis, 94220 Charenton le Pont (FR)
(74) Mandataire: Burtin, Jean-François
(86) Numéro de dépôt international: PCT/FR1999/000680
(87) Numéro de publication internationale: WO 1999/048477

(56) Documents cités:
- EP-A- 0 785 211
- EP-A- 0 785 212
- WO-A-92/08730
- WO-A-94/06437

## Description

La présente invention se rapporte au domaine de la chimie thérapeutique et plus spécialement à la réalisation de nouvelles formes galéniques destinées à être appliquées sur la peau.

La présente invention a plus particulièrement pour objet des préparations galéniques dont le principe actif est un progestatif de synthèse, destinées à être appliquées sur la peau afin d'obtenir un effet systémique hormonal chez la femme avant et après la ménopause.

Ainsi, l'invention concerne une composition hormonale topique à effet systémique.

Les progestatifs de synthèse ainsi que la progestérone naturelle sont habituellement utilisés par voie orale pour la correction des insuffisances lutéales chez la femme non ménopausée et pour le traitement hormonal substitutif de la ménopause.

Le brevet français 2.271.833 décrit notamment des compositions hormonales pour la correction des carences progestatives chez la femme préménopausée ou ménopausée, destinées à l'administration par voie orale.

Cependant, la voie orale n'est pas sans présenter quelques inconvénients pour la progestérone naturelle, mais aussi pour les progestatifs de synthèse. D'une part, elle oblige à administrer des doses assez importantes afin de compenser la dégradation du principe actif lors du passage à travers l'intestin et dans le foie (effet dit « de premier passage »). D'autre part, elle ne donne pas des taux plasmatiques constants au cours du temps puisque la prise orale est suivie par un pic plasmatique au cours duquel les concentrations sanguines sont transitoirement élevées.

La progestérone naturelle est parfois administrée par voie percutanée. Cette voie produit seulement des effets locaux, mais elle ne permet pas d'obtenir une imprégnation à distance des tissus cibles, notamment de la muqueuse utérine. La raison en est la dégradation rapide de l'hormone par les enzymes du tissu sous-cutané, qui ne permet pas d'atteindre des taux plasmatiques suffisants pour engendrer une action hormonale systémique.

Beaucoup de progestatifs de synthèse présentent le même inconvénient et ne peuvent être utilisés par voie percutanée pour obtenir un effet systémique. Seul fait exception l'acétate de noréthistérone administré dans des patchs.

La fonction de barrière protectrice de la peau contre les agressions extérieures la rend difficilement perméable vis-à-vis de nombreuses substances et ne laisse pénétrer les molécules médicamenteuses que sous certaines conditions : taille et nature de la molécule, solubilité, stabilité, nature du véhicule contenant la molécule, etc.
Ainsi, la libération d'un principe actif à partir d'un véhicule et sa pénétration à travers la peau jusque vers la circulation sanguine ou lymphatique dépend de nombreux paramètres physico-chimiques et/ou physiologiques.

Dans la présente invention, la nature même du principe actif (progestatif de synthèse) présente le principal obstacle à la pénétration percutanée : le problème majeur qui se pose est sa faible diffusion à travers l'épiderme du fait de son caractère lipophile. Le choix du véhicule utilisé dans les compositions aura donc une grande importance sur la pénétration percutanée et l'activité thérapeutique de la molécule.
Ainsi, les compositions topiques selon l'invention permettent un effet systémique par optimisation du passage percutané d'un progestatif de synthèse dérivé de la 19-nor progestérone.

Les compositions topiques selon l'invention contiennent à titre de principe actif un progestatif de synthèse dérivé de la 19-nor progestérone et des excipients permettant d'optimiser le passage percutané du principe actif

La présente invention a plus spécifiquement pour objet une composition hormonale topique à effet systémique telle que définie dans la revendication 1.

Les compositions conformes à l'invention pourront donc se trouver sous forme de gel ou de gel filmogène.

Le progestatif dérivé de la 19-nor progestérone utilisé dans la présente invention est le nomégestrol et/ou un de ses esters ou éthers.
Un exemple d'éther de nomegestrol est l'éther tétrahydropyranique de nomegestrol.
Un exemple d'ester de nomegestrol est l'acétate de nomegestrol, qui est un progestatif de synthèse actif par voie orale, dont l'action réside dans la correction des troubles gynécologiques provoqués par l'insuffisance d'hormones lutéinisantes.

Administré à l'aide des compositions conformes à l'invention, l'acétate de nomégestrol est capable de traverser la peau et de passer dans la circulation sanguine pour donner des taux plasmatiques détectables à l'aide des méthodes utilisées pour le doser dans les milieux biologiques. Les concentrations plasmatiques observées se maintiennent en plateau après l'application cutanée en raison de l'effet réservoir de la peau.

Les taux plasmatiques d'acétate de nomégestrol obtenus avec les compositions qui font l'objet de la présente invention sont à même de créer un effet hormonal sur les tissus situés à distance du lieu d'application et notamment sur l'endomètre.

L'acétate de nomégestrol ainsi administré de façon réitérée génère une action thérapeutique lorsqu'il est donné à des femmes non ménopausées souffrant d'une symptomatologie liée à une insuffisance en progestérone ou à des femmes ménopausées soumises à une estrogénothérapie substitutive.

Selon la présente invention, le nomegestrol ou un de ses esters ou éthers est présent en une quantité variant de 0,05 à 1 % en poids de la composition totale. De préférence, le nomegestrol ou un de ses esters ou éthers est présent en une quantité variant de 0,1 à 0,8 % en poids de la composition totale. Les compositions topiques à effet systémique préférées selon l'invention, sont celles contenant une quantité de nomegestrol ou d'un de ses esters ou éthers de 0,4 % en poids de la composition totale.

Les agents solubilisants et les agents promoteurs d'absorption ont des modes d'action qui sont différents mais permettent tous les deux de favoriser la pénétration à travers la peau du principe actif.
Les agents solubilisants, par une action sur l'activité thermodynamique de la molécule active, améliorent la solubilité du principe actif et modifient son affinité pour la peau.

Les agents promoteurs d'absorption, par des modifications au niveau de la structure de la barrière cutanée diminuent la résistance à la diffusion.

Cependant, il n'y a pas de relation directe entre amélioration de la solubilité du principe actif dans le véhicule et augmentation de son passage percutané. En effet, l'utilisation d'agents améliorant la solubilité du principe actif augmente également son affinité pour le véhicule et donc diminue le plus souvent sa diffusion à travers la peau.
Ainsi, pour que la solubilité du principe actif dans un véhicule puisse être totale, il doit y avoir une certaine affinité pour celui-ci ; néanmoins, elle ne doit pas être trop importante afin que le partage du principe actif s'oriente vers sa diffusion à travers la peau.

Selon la présente invention, des exemples d'agents solubilisants appropriés sont l'eau, les alcools, le propylèneglycol, le polyéthylène glycol, le polyéthylène 20 sorbitane mono-oléate (commercialisé par exemple sous la dénomination Polysorbate 80 DF), un glycéride en C₈/C₁₀ polyoxyéthyléné glycosylé (commercialisé par exemple sous la dénomination Labrasol®) ou leurs mélanges.

On emploie généralement, à titre d'agent solubilisant, un mélange de solvants ou d'agents solubilisants précités, qui, par une synergie d'action, est plus efficace que l'un d'eux utilisé seul.
De préférence, l'agent solubilisant est choisi dans le groupe constitué par l'eau, les alcools, le propylèneglycol, un glycéride en C₈/C₁₀ polyoxyéthyléné glycosylé ou leurs mélanges.
Ainsi, on pourra par exemple employer, à titre d'agent solubilisant, un mélange binaire éthanol à 95° / eau, dans lequel le pourcentage d'éthanol à 95° varie de 30 à 50 %, et plus particulièrement un mélange binaire éthanol à 95° /eau dans lequel le pourcentage d'éthanol à 95° est de 45%.

Cependant, des exemples particulièrement préférés d'agent solubilisant appropriés pour la composition topique à effet systémique selon l'invention, sont :
- un mélange ternaire éthanol à 95° / eau / propylèneglycol, dans lequel le pourcentage d'éthanol à 95° varie de 30 à 50 %, celui d'eau de 30 à 60 %, et celui de propylèneglycol de 2 à 20 % ; de préférence, le pourcentage d'éthanol à 95° est de 45 %, celui d'eau de 45 %, et celui de propylèneglycol de 8 %,
- un mélange quaternaire éthanol à 95° / eau / Labrasol® / propylèneglycol, dans lequel le pourcentage d'éthanol à 95 ° varie de 30 à 50 %, celui d'eau de 30 à 60 %, celui de Labrasol® de 3 à 7 % et celui de propylèneglycol de 2 à 20 % ; de préférence, le pourcentage d'éthanol à 95° est de 45 %, celui d'eau de 33,5 %, celui de Labrasol® de 5 % et celui de propylèneglycol de 15 % ;

Parmi les substances considérées comme des agents promoteurs d'absorption, ou « enhancers », les plus utilisés sont les dérivés du glycol, des sulfoxides, des tensioactifs, des acides gras et des dérivés terpéniques.

A titre d'exemple d'agents promoteurs d'absorption, on pourra citer l'acide oléique, l'alcool oléique, un triglycéride des acides caprique et caprylique (par exemple, celui commercialisé sous la dénomination Miglyol 812®), le myristate d'isopropyle, le dipelargonate de propylèneglycol, le 2n-nonyl-1,3-dioxolane, le myristate d'octyl dodécyle, l'isopropylidène glycérol (par exemple celui commercialisé sous la dénomination Solkétal), l'α-tocophéryl polyéthylèneglycol 1000 succinate (par exemple celui commercialisé sous la dénomination Vitamine E TPGS), l'éther monoéthylique du diéthylène glycol (par exemple celui commercialisé sous la dénomination Transcutol®).

L'agent promoteur d'absorption convenant plus particulièrement dans la présente invention est choisi dans le groupe constitué par l'isopropylidèneglycérol, l' α-tocophéryl polyéthylèneglycol 1 000 succinate et l'éther monoéthylique du diéthylène glycol.
L'agent promoteur d'absorption cependant préféré est l'isopropylidèneglycérol.

Les formes envisagées pour assurer la pénétration percutanée du principe actif seront soit des gels, soit des préparations gélifiées occlusives.
Le choix des agents gélifiants et des agents filmogènes est également important dans les compositions selon l'invention.
Les agents gélifiants sont des substances qui épaississent et modifient la viscosité d'un véhicule liquide constituant ainsi un réseau colloïdal tridimensionnel, le gel.
Il existe plusieurs sortes d'agents gélifiants : les agents gélifiants naturels (minéraux, végétaux, animaux), les agents synthétiques et les agents semi-synthétiques.

Des exemples d'agents gélifiants naturels sont la gomme guar, les extraits d'algues (les alginates, les carraghénates, la gélose), les polysaccharides (la gomme xanthane, la gomme arabique, la gomme adragante), les amidons, les pectines, etc.

Des exemples d'agents gélifiants synthétiques ou semi-synthétiques sont les dérivés cellulosiques, notamment ceux obtenus par estérification ou par éthérification de la cellulose, et les dérivés acryliques. Dans la catégorie des dérivés acryliques, on classe les carbomères, les polycarbophiles, les acrylates.
Dans la présente invention, l'agent gélifiant est choisi dans le groupe constitué par les dérivés cellulosiques et les dérivés acryliques.

Parmi les dérivés cellulosiques, on trouve :
- les méthylcelluloses (Methocel, Metolose),
- les éthylcelluloses (Ethocel, Aquacoat®)
- les hydroxypropylméthylcelluloses (Kenal Methocel, Hypromelose),
- les hydroxyéthylcelluloses (Cellosize, Natrosol),
- les hydroxypropylcelluloses (Klucel),
- les carboxyméthylcelluloses sous forme sodique ou calcique (Akucell, Nymcel Tylose CB),

Le choix d'un polymère se fait dans la gamme Métolose, de la firme Shin Etsu. Pour chacun de ces types, il existe différents degrés (ou grades) en fonction des substituants et du degré de substitution qui donnent aux solutions de polymères des viscosités différentes. Il existe une classification des celluloses en fonction de leur potentiel adhésif. Le choix du grade est important car le pouvoir adhésif du dérivé cellulosique varie en fonction de celui-ci.
Selon la présente invention, un dérivé cellulosique particulièrement approprié est l'hydroxypropylméthylcellulose, et tout particulièrement l'hydroxypropylméthylcellulose de grade 60 SH 4000. En effet, le grade 60 SH présente les spécifications les mieux adaptées : une bonne solubilité dans les solvants organiques et une résistance élevée aux électrolytes. Il permet également d'obtenir des gels transparents.
Parmi les dérivés acryliques on retiendra notamment, selon la présente invention, les carbomères, et notamment ceux commercialisés sous les dénominations Carbopol ® ou Synthalen ®.

Les carbomères donnent des formulations stables dans le temps et confèrent à la formulation des propriétés rhéologiques reproductibles du fait de leur nature synthétique.
L'existence de différents degrés ou grades tient à la différence du poids moléculaire, au degré de réticulation, à la nature des arrangements moléculaires et au solvant de polymérisation.
Ainsi, parmi les différents grades de carbomère, on pourra citer ceux commercialisés par la société Goodrich sous les dénominations Carbopol 974 P ®, Carbopol 980 ®, Carbopol 1382 ® et Carbopol 2020 ®, ou des produits similaires comme les Synthalen de 3 V France, tels quels (Synthalen K, L, M) ou préneutralisés, comme par exemple les Synthalen PNC ®.
Cependant, selon la présente invention, les carbomères commercialisés sous les dénominations Carbopol 980®, Carbopol 1382® et Synthalen K® sont particulièrement appropriés et présentent des avantages non négligeables, car ils se fluidifient au contact des électrolytes de la peau et évitent ainsi un dépôt de polymère qui risque de faire obstacle au passage du principe actif

Les agents filmogènes utilisés sont ceux qui sont employés pour réaliser des solutions d'enrobage, de pelliculage car ils sont issus de l'industrie alimentaire ou biomédicale pour la plupart d'entre eux et permettent d'envisager une application chez l'homme.
Ces agents filmogènes peuvent être classés en différents groupes en fonction de leur solubilité. Quel que soit l'agent filmogène, la qualité du gel filmogène obtenu ou de la solution filmogène obtenue dépend du pourcentage d'agent filmogène, de la nature du solvant, de la présence et de la nature de l'agent plastifiant.

Selon la présente invention, l'agent filmogène est choisi dans le groupe constitué par les dérivés cellulosiques, les dérivés méthacryliques et les dérivés de la polyvinylpyrrolidone.

Parmi les dérivés cellulosiques, on pourra citer :
- l'acétate succinate d'hydroxypropylméthylcellulose, et notamment celui commercialisé par la société Seppic sous la dénomination Aqoat AS-LF®,
- une dispersion aqueuse d'acétophtalate de cellulose contenant 70 % d'eau, 23 % d'acétophtalate de cellulose et 7 % de poloxamer, et notamment celle commercialisée par la société Seppic sous la dénomination Aquacoat CPD®,
- une dispersion aqueuse d'éthylcellulose, d'alcool cétylique et de lauryl sulfate de sodium, et notamment celle commercialisée par la société Seppic sous la dénomination Aquacoat ECD 30®,
- l'éthylcellulose.

Parmi les dérivés méthacryliques, on pourra citer :
- une dispersion aqueuse d'un copolymère anionique d'acide méthacrylique et d'éthyle acrylate (type C), notamment celle contenant 30 % de copolymère sec, 0,7 % de lauryl sulfate de sodium et 2,3 % de Polysorbate 80 NF, et commercialisée sous la dénomination Eudragit L30 D55® (Rohm et Haas),
- un copolymère d'acide acrylique et d'ester méthacrylique (type A), notamment celui commercialisé sous la dénomination Eudragit RL 100® (Rohm et Haas).

Parmi les dérivés de la polyvinylpyrrolidone, on pourra citer :
- une povidone, de formule (C₆H₉NO)ₙ dont le poids moléculaire est de l'ordre de 360 000, commercialisée sous la dénomination Kollidon 90®
- le copolymère polyvinylpyrrolidone / acétate de vinyle 64, de formule (C₆H₉NO)ₙ x (C₄H₆O₂)ₘ dont le poids moléculaire est : (111,1)ₙ x (86, 1)ₘ.
- les homopolymères d'alcool polyvinylique

Dans la présente invention, le dérivé cellulosique particulièrement approprié est l'acétate succinate d'hydroxypropylméthylcellulose, le dérivé méthacrylique particulièrement approprié est une dispersion aqueuse d'un copolymère anionique d'acide méthacrylique et d'éthyle acrylate, et le dérivé de la polyvinylpyrrolidone particulièrement approprié est une povidone.

Les compositions hormonales topiques à effet systémique selon l'invention peuvent contenir en outre d'autres excipients qui sont des agents complexants, des agents neutralisants tels que l'édétate disodique (EDTA), la triéthanolamine (TEA) et/ou des agents plastifiants tels que le phtalate de diéthyle, la triacétine.

Une composition hormonale topique selon l'invention particulièrement appropriée est une composition sous forme de gel ou de gel filmogène, présentant une quantité de nomégestrol ou d'acétate de nomegestrol de 0,4 % en poids de la composition totale, un pH compris entre 6 et 7, et une viscosité comprise entre 1000 et 2000 mPas.
Le procédé de préparation des compositions à effet systémique selon l'invention diffère selon la nature même des compositions que l'on désire obtenir, à savoir un gel, un gel filmogène ou une solution filmogène.

### • PROCEDE DE PREPARATION DES GELS

De même, lors de la préparation de compositions sous forme de gel, le mode de préparation ne sera pas tout à fait le même selon la nature de l'agent gélifiant utilisé. Ainsi, lors de la préparation des gels, on distinguera, en ce qui concerne l'agent gélifiant, les dérivés acryliques synthétiques des dérivés cellulosiques.

### - Préparation à partir de dérivés acryliques

Les étapes importantes de la préparation d'un gel sont la dispersion de l'agent gélifiant dans l'agent solubilisant (dispersion de laquelle dépendra beaucoup la qualité de la préparation obtenue), l'agitation, l'hydratation, le gonflement et enfin la gélification.

### Dispersion et agitation: mouillage

Le dérivé acrylique est mis en suspension sous agitation dans le solvant (agent solubilisant). L'agitation doit être modérée, faute de quoi le polymère acrylique se dégrade par cisaillement et perd de son efficacité.

### Hydratation et gonflement des polymères

Afin d'éviter la formation d'agglomérats partiellement hydratés, il est recommandé d'incorporer les polymères en les tamisant, pour faciliter la mouillabilité et l'hydratation de la poudre et leur permettre de se déployer en réseau. On favorise cette étape en réalisant un mouillage de la poudre au préalable, dans le solvant le plus polaire en cas de système solvant.

### Gélification : neutralisation de la dispersion obtenue

Le pH d'une telle suspension est proche de 3 (ce pH est fonction de la concentration en polymère, donc en groupements carboxyliques). On utilise des bases minérales comme les hydroxydes de sodium, de potassium ou d'ammonium lorsque les solvants de la formulation sont aqueux et des bases organiques comme des amines (triéthanolamine, trométhamine ou TRIS etc...) lorsqu'ils sont peu ou pas polaires. L'ajout de ces agents provoque un épaississement spontané par formation des sels de résines de polymères, solubles dans l'eau.
Un exemple de préparation d'un gel dont l'agent gélifiant est un dérivé acrylique, se définit en ce que :
- on solubilise l'acétate de nomégestrol et l'EDTA dans le système solvant eau / éthanol à 95° / propylèneglycol en agitant à 300 t/min (# 30 min) ;
- on disperse le polymère acrylique par petites fractions dans la solution de principe actif en agitant à 100 t/min ;
- on laisse gonfler le polymère acrylique pendant 2 heures sous agitation à 200 t/min ;
- on neutralise la dispersion par de la triéthanolamine dissoute dans une fraction d'eau prélevée sur la quantité à incorporer dans la formulation ; on réduit l'agitation à 100 t/min durant la neutralisation pour éviter l'incorporation de bulles d'air ;
- on agite 30 min à 150 t/min pour homogénéiser le gel obtenu.

### - Préparation à partir de dérivés cellulosiques

Les gels formulés à base de dérivés cellulosiques n'ont pas besoin d'être neutralisés, mais il sera quelquefois nécessaire d'ajuster leur pH au moyen d'amines organiques ou d'hydroxydes minéraux, selon la nature du solvant de formulation.
La viscosité obtenue dépend de la nature et de la quantité de dérivé cellulosique utilisé.

Un exemple de préparation d'un gel dont l'agent gélifiant est un dérivé cellulosique, se définit en ce que :
- on solubilise l'acétate de nomégestrol et l'EDTA dans le système solvant eau / éthanol 95° / propylèneglycol en agitant à 300 t/min (# 30 min) ;
- on disperse le polymère cellulosique par petites fractions dans la solution de principe actif en agitant à 100 t/min ;
- on laisse gonfler le polymère cellulosique 1 heure sous agitation à 250 t/min ;
- on ajuste le pH, si nécessaire, par la triéthanolamine dissoute dans l'eau en agitant à 100 t/min ;
- on agite 30 min à 150 t/min pour homogénéiser le gel obtenu.

### • GELS FILMOGENES (ou « GELS FILMANTS ») ET SOLUTIONS FILMOGENES (ou « SOLUTIONS FILMANTES »)

De telles formes sont envisagées, car, lors de leur application sur la peau, elles forment, après séchage une sorte de film occlusif, suffisant pour augmenter l'hydratation de la peau et créer de nouveaux sites de passage, et améliorant ainsi la diffusion du principe actif qu'elles contiennent. La forme obtenue devra néanmoins pénétrer ou sécher rapidement tout en laissant un toucher agréable et non collant.
Les agents filmogènes utilisés dans la présente invention sont en général ceux utilisés pour la réalisation de solutions d'enrobage pour comprimés.

### • PROCEDE DE PREPARATION DES SOLUTIONS FILMOGENES

Comme pour les gels, lors de la préparation des compositions sous forme de solution filmogène, le mode de préparation sera différent selon la nature de l'agent filmogène utilisé.

### - Préparation à partir d'un agent filmogène solide :

Les étapes de la préparation sont :
- Solubilisation d'un agent plastifiant et du principe actif dans le mélange solvant : le temps d'agitation du mélange contenant le plastifiant et le principe actif doit être suffisant pour obtenir une solution.
- Dispersion et solubilisation de l'agent filmogène :

La dispersion doit se faire par petites fractions, sous agitation vive. L'agitation se poursuit jusqu'à complète solubilisation de l'agent filmogène. La neutralisation de la solution filmogène se fait si nécessaire en fin de fabrication, sous agitation réduite.

Un exemple de préparation d'une solution filmogène dont l'agent filmogène est solide, se définit en ce que :
- on agite les quantités d'éthanol, d'eau et de propylèneglycol nécessaires à la formulation à 250 t/min pendant 10 min ;
- on solubilise l'EDTA et l'acétate de nomégestrol dans le mélange obtenu ;
- on ajoute l'agent plastifiant et on agite à 250 t/min pendant 30 min ;
- on disperse l'agent filmogène par petites fractions en gardant la même agitation, jusqu'à sa complète solubilisation ; et on poursuit l'agitation 1 heure ;
- on ajuste le pH à l'aide d'une solution de triéthanolamine dissoute dans une petite quantité d'eau, prélevée sur la quantité d'eau à incorporer à la formulation, en réduisant l'agitation à 100 t/min ; on homogénéise la solution obtenue pendant 30 min.

### - Préparation à partir d'un agent filmogène en dispersion aqueuse

Les étapes de la préparation sont :
- Solubilisation et plastification de l'agent filmogène
- Incorporation du mélange renfermant le principe actif et les autres excipients par petites fractions, sous agitation vive

La neutralisation se fait en fin de fabrication sous agitation réduite.

Un exemple de préparation d'une solution filmogène dont l'agent filmogène est en dispersion aqueuse, se définit en ce que :
- on mélange à 250 t/min l'eau et un agent plastifiant ; on agite pendant 30 min ;
- on ajoute par petites fractions la dispersion d'agent filmogène en agitant à 250 t/min, jusqu'à obtention d'une solution homogène de la dispersion ; on poursuit l'agitation 1 heure ;
- indépendamment, on solubilise l'EDTA et l'acétate de nomégestrol dans le mélange éthanol et propylèneglycol ; on agite jusqu'à totale dissolution ;
- on ajoute par petites fractions la solution alcoolique de principe actif dans la solution aqueuse, sous agitation de 250 t/min; on agite la solution obtenue pendant 1 heure pour l'homogénéiser ;
- on neutralise la solution par la triéthanolamine dissoute dans l'eau, en réduisant l'agitation ; on homogénéise la solution obtenue pendant 30 min.

### • PROCEDE DE PREPARATION DES GELS FILMOGENES OU FILMS GELIFIES

Les gels filmogènes sont obtenus par gélification des solutions filmogènes.
On commence par préparer séparément deux solutions :
- une solution aqueuse renfermant un plastifiant solubilisé, dans laquelle on solubilise totalement l'agent filmogène sous agitation vive ;
- une solution alcoolique contenant les autres excipients de la formulation et dans laquelle on solubilise le principe actif ; on disperse et laisse gonfler l'agent gélifiant.
Puis, on mélange la solution alcoolique dans la solution aqueuse et on gélifie la solution par la triéthanolamine.

Un exemple de préparation d'un gel filmogène se définit en ce que :
- on solubilise l'agent plastifiant dans l'eau ; on agite pendant 30 min à 250 t/min ;
- on disperse l'agent filmogène et on agite à 250 t/min jusqu'à totale solubilisation (dans le cas d'un agent filmogène solide) ou homogénéité de la dispersion ; on prolonge l'agitation pendant 1 heure ;
- indépendamment, on solubilise l'EDTA et l'acétate de nomégestrol dans le mélange constitué de propylèneglycol et d'éthanol ; on disperse l'agent gélifiant choisi et on laisse gonfler 2 heures en agitant à 150 t/min ;
- on mélange la solution alcoolique dans la solution aqueuse et on agite 1 heure à 150 t/min ;
- on neutralise par la triéthanolamine dissoute dans l'eau, en réduisant la vitesse d'agitation à 100 t/min ; on homogénéise le gel obtenu en agitant 30 min.

### METHODE D'EVALUATION DU PASSAGE PERCUTANE DU PRINCIPE ACTIF

L'efficacité de la composition topique selon l'invention est évaluée en démontrant que le principe actif qu'elle contient diffuse à travers la peau et est résorbé dans la microcirculation en quantité suffisante pour obtenir l'effet thérapeutique souhaité.

Dans la présente invention, le passage percutané de l'acétate de nomégestrol est évalué par mesure de la radioactivité en utilisant une molécule marquée au carbone 14. La méthode d'évaluation du passage du principe actif utilisant les produits radiomarqués permet de détecter de faibles taux de principe actif, ce qui représente un avantage considérable si l'on considère les faibles quantités qui diffusent à travers la peau.

La peau utilisée au cours des différents essais d'évaluation du passage percutané du principe actif, provient d'interventions de chirurgie plastique abdominale sur des sujets féminins, âgés de 40 à 45 ans. La peau de la femme est débarrassée de l'excès de tissu adipeux, nettoyée puis conservée au congélateur à - 70°C.

Les compositions topiques conformes à l'invention sont destinées à être appliquées principalement sur la peau de l'abdomen, des bras, des cuisses, etc.

### PARTIE EXPERIMENTALE

### • EXEMPLE I

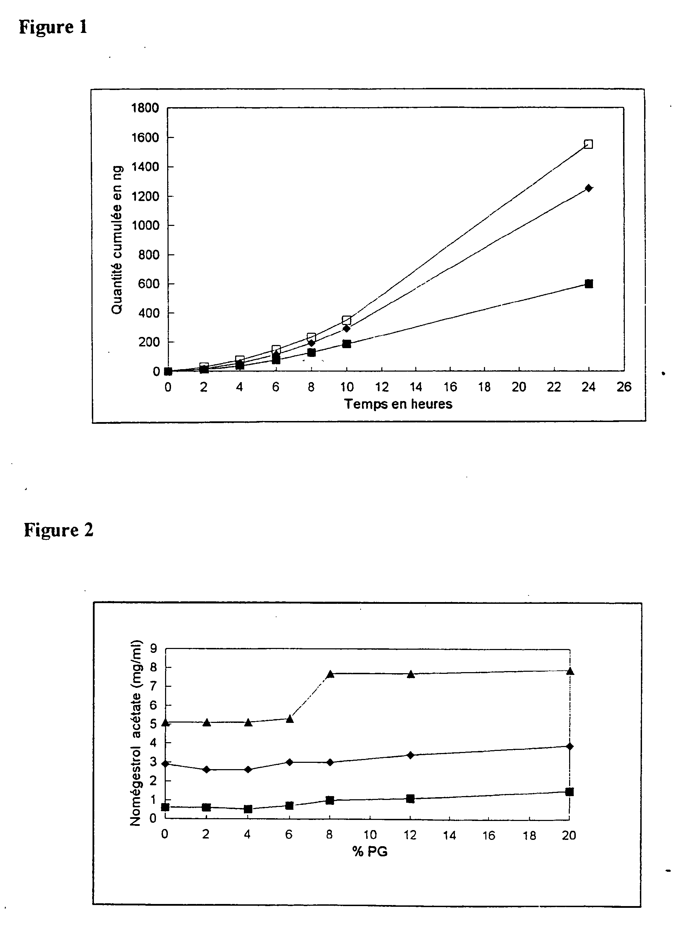

La figure 1 illustre le passage percutané du principe actif acétate de nomegestrol (AcN) en fonction de différentes quantités d'acétate de nomegestrol dans les compositions conformes à l'invention.
Les symboles ■, ◆, et □ de la figure 1 représentent :

| | | |
|---|---|---|
| ■ Gel 0,11 % d'AcN | ◆ Gel 0,4 % d'AcN | □ Gel 0,8 % d'AcN |

Ces compositions se trouvent sous forme de gel et leurs formulations sont présentées dans le tableau 1 ci-dessous :

**Tableau 1**

| *FORMES* | *GELS* | | |
|---|---|---|---|
| FORMULATIONS (en %) | | | |
| Nomégestrol Acetate | 0,40 | 0,80 | 0,11 |
| Propylèneglycol | 6,00 | 6,00 | 3,00 |
| Transcutol® | 5,00 | 5,00 | - |
| Carbopol 1342® | 0,50 | 0,50 | - |
| Carbopol 940® | / | / | 0,75 |
| EDTA | 0,05 | 0,05 | 0,05 |
| Triéthanolamine (TEA) | 0,30 | 0,30 | 0,30 |
| Eau déminéralisée | 42,75 | 42,35 | 45,79 |
| Ethanol | 45,00 | 45,00 | 50,00 |
| pH (à 1 %) | 6,7 | 6,5 | 6,7 |
| NOMEGESTROL ACETATE (mg/g) | 0,41 | 0,4 | 0,403 |

Le passage percutané du principe actif est évalué par mesure de la quantité cumulée de principe actif en fonction du temps. La quantité de principe actif cumulée représente la totalité de la diffusion du principe actif à travers la peau sur une période déterminée (24 ou 48 h). Dans cet exemple, elle est exprimée en ng.

La figure 1 indique clairement que les résultats de diffusion les plus faibles sont obtenus avec le gel à 0,11 % d'AcN.

Ce gel à 0,11 % a été testé dans des essais cliniques préliminaires : cf. exemple IV. Ainsi, il a été établi que ce gel, malgré ses moins bons résultats, permettait tout de même d'obtenir un effet de passage systémique.

### • EXEMPLE II

### Etude de la solubilité de l'acétate de nomégestrol (AcN)

### 1) - a) Dans un mélange binaire éthanol à 95° / eau

On détermine le système de solvant en mélange hydroalcoolique qui est le plus efficace.

**Tableau 2 : Solubilité de l'acétate de nomégestrol en fonction du pourcentage d'éthanol à 95°**

| % d'éthanol à 95° | Solubilité de l'acétate de nomégestrol en mg/ml |
|---|---|
| 0 | 0,056 |
| 10 | 0,070 |
| 20 | 0,113 |
| 30 | 0,683 |
| 40 | 2,820 |
| 50 | 7,330 |
| 60 | 17,850 |
| 70 | 24,850 |
| 80 | 29,500 |
| 90 | 26,600 |
| 100 | 32,850 |

En mélange hydroalcoolique, la solubilité augmente avec le pourcentage d'alcool. Le profil de solubilité montre que celui-ci est assez faible jusqu'à 40 % d'alcool, puis augmente fortement entre 40 et 80 %. Or, le pourcentage d'alcool autorisé pour les formes à usage topique est limité. Dans ces limites, le système solvant le plus efficace pour solubiliser l'acétate de nomégestrol se situe entre 40 et 60 % d'alcool.

### - b) Dans un mélange ternaire éthanol à 95° / eau / propylèneglycol

On a déterminé l'effet d'un mélange ternaire de solvants, éthanol / eau (45:55) / propylèneglycol sur la solubilité de l'acétate de nomégestrol.
On a examiné également la possibilité de diminuer la proportion d'alcool du solvant, grâce à ce mélange ternaire, tout en conservant une solubilité similaire ; on choisit pour cela l'influence du propylèneglycol sur la solubilité dans les systèmes éthanol / eau (40:60 et 30:70).

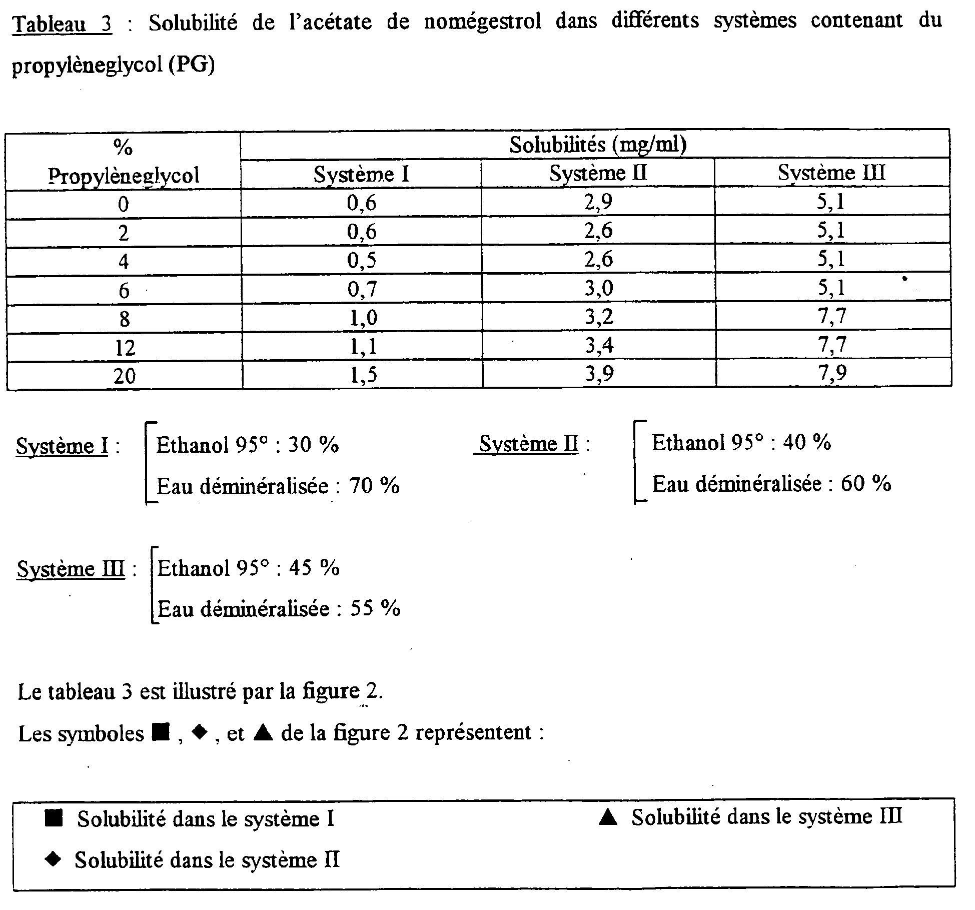

En mélange ternaire eau / éthanol / propylèneglycol, la solubilité du principe actif est améliorée pour les proportions de 8 % de propylèneglycol pour un mélange à 45 % d'alcool. C'est pour ce système que l'on obtient la meilleure solubilité du principe actif. Le propylèneglycol agit en synergie avec l'alcool, sur la solubilité de l'acétate de nomégestrol.

### - c) Dans le mélange éthanol à 95° / eau / Labrasol / propylèneglycol

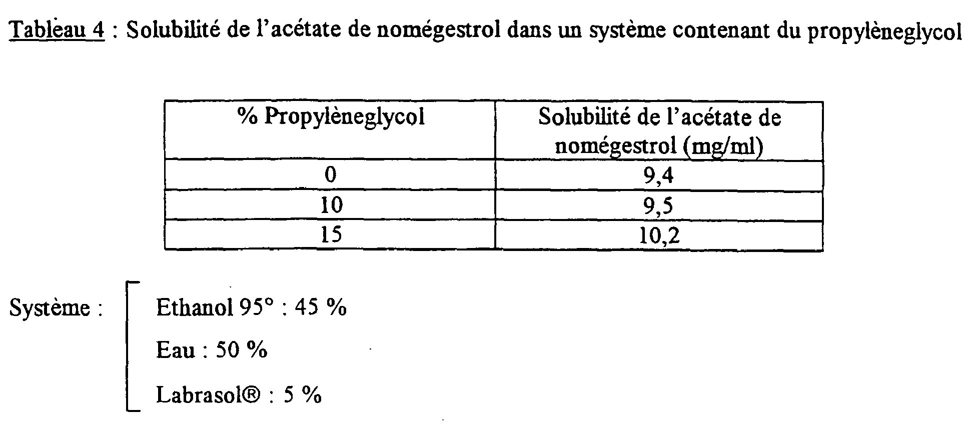

En utilisant le Labrasol® seul à 5 % sans le propylèneglycol, la solubilité augmente par rapport aux résultats obtenus avec le propylèneglycol. On améliore encore cette solubilité en associant propylèneglycol et Labrasol®.

### 2) - a) Dans un mélange éthanol à 95° / eau / propylèneglycol / Solkétal

**Tableau 5 : Solubilité de l'acétate de nomégestrol dans un mélange hydroalcoolique contenant du propylèneglycol et/ou du Solkétal**

| % Propylèneglycol | % Solkétal | Solubilité de l'acétate de nomégestrol (mg/ml) |
|---|---|---|
| 0 | 3 | 6,7 |
| 0 | 8 | 8,6 |
| 8 | 0 | 7,7 |
| 8 | 3 | 10,7 |

La solubilité de l'acétate de nomégestrol dans le mélange solvant hydroalcoolique en présence de 8 % de Solkétal est supérieure à celle obtenue en présence de 8 % de propylèneglycol seul.
L'association des deux substances propylèneglycol Solkétal augmente sensiblement la solubilité dans le mélange solvant hydroalcoolique, dans la proportion de 8 % de propylèneglycol / 3% de Solkétal.

### - b) Dans un mélange éthanol à 95° / eau / propylèneglycol / vitamine E TPGS

**Tableau 6 : Solubilité de l'acétate de nomégestrol dans un mélange hydroalcoolique contenant du propylèneglycol et/ou de la vitamine E TPGS**

| % Propylèneglycol | % Vitamine E TPGS | Solubilité de l'acétate de nomégestrol (mg/ml) |
|---|---|---|
| 0 | 3 | 7,65 |
| 0 | 8 | 11,10 |
| 8 | 0 | 7,70 |
| 8 | 3 | 12,50 |

La solubilité de l'acétate de nomégestrol est améliorée en présence de vitamine E TPGS seule, par rapport au propylèneglycol, pour une même proportion de 8 %. Incorporée à 3 %, elle donne déjà des résultats équivalents au propylèneglycol utilisé à 8 %.
Toutefois, on obtient une solubilité encore meilleure lorsque l'on associe ces deux substances dans la proportion de 8 % de propylèneglycol / 3 % de vitamine E TPGS.

### - c) Dans un mélange éthanol à 95° / eau / propylèneglycol / Transcutol®

**Tableau 7 : Solubilité de l'acétate de nomégestrol dans un mélange hydroalcoolique contenant du propylèneglycol et/ou du Transcutol®**

| % Propylèneglycol | % Transcutol ® | Solubilité d'acétate de nomégestrol (mg/ml) |
|---|---|---|
| 8 | 0 | 7,70 |
| 8 | 3 | 7,95 |
| 0 | 8 | 10,70 |
| 3 | 8 | 10,60 |

La solubilité de l'acétate de nomégestrol est améliorée en présence de 8 % de Transcutol® seul, par rapport au propylèneglycol à la même proportion. En associant ces deux substances, on obtient une même solubilité si l'on utilise la proportion de 8 % de Transcutol / 3 % de propylèneglycol.
La proportion inverse ne permet pas d'améliorer la solubilité du principe actif par rapport à celle obtenue dans le propylèneglycol seul.

### Conclusion:

Les mélanges hydroalcooliques contenant :
- 8 % de propylèneglycol et 3 % de Solkétal,
- ou 8 % de propylèneglycol et 3 % de vitamine E TPGS,
- ou 3 % de propylèneglycol et 8 % de Transcutol®,
sont particulièrement appropriés pour une bonne solubilité du principe actif

### • EXEMPLE III

### 1/ Etude des formulations sous forme de gels

Parmi les substances choisies pour leurs qualités d'agents promoteurs d'absorption, le Solkétal et la vitamine E TPGS sont particulièrement appropriés puisqu'ils s'avèrent également capables d'améliorer la solubilité de l'acétate de nomégestrol en mélange hydroalcoolique et propylèneglycol. On a étudié l'action promotrice des trois agents promoteurs en les incorporant dans des formulations contenant un gel hydroalcoolique à 45 % d'alcool et renfermant 8 % de propylèneglycol et 3 % de l'agent promoteur. Ces formulations sous forme de gels sont testées en passage percutané.
Les gels retenus répondent aux spécifications de pH, viscosité, titre et aspect.

Les quatre gels étudiés sont dénommés « G36-264, G36-276, G32-104 et G37-113 » et leurs formulations sont présentées dans le tableau 8 ci-dessous :

**Tableau 8 :**

| *FORMES* | *GELS* | | | |
|---|---|---|---|---|
| Références | G36-264 | G36-276 | G32-104 | G37-113 |
| | | | référence | référence |
| Formulations en % | | | | |
| Nomégestrol Acetate | 0,4 | 0,4 | 0,4 | 0,4 |
| Propylèneglycol | 8 | 8 | 8 | 8 |
| Transcutol® | / | / | 3 | 3 |
| Solkétal | 3 | / | / | / |
| Vit E TPGS | / | 3 | / | / |
| HPMC 60SH4000 | / | / | / | / |
| Carbopol 1382® | / | / | 0,5 | / |
| Carbopol 980® | 0,5 | 0,5 | / | 0,6 |
| EDTA Edétate disodique | 0,05 | 0,05 | 0,05 | 0,05 |
| TEA | 0,4 | 0,3 | 0,3 | 0,25 |
| Kollidon 90® | / | / | / | / |
| Aqoat AS-LF® | / | / | / | / |
| Eudragit L30D55® | / | / | / | / |
| Phtalate de diéthyle | / | / | / | / |
| Ethanol 95 | 45 | 45 | 45 | 45 |
| Eau déminéralisée | 42,65 | 42,75 | 42,75 | 42,85 |
| pH solution à 1 % | 6,9 | 6,92 | 6,6 | 6,37 |
| Viscosité mPa.s | 1150 | 1020 | 1400 | 1400 |
| Titre AcN(%) | 0.41 | 0,4 | 0,403 | 0,393 |

La principale différence dans la composition de ces gels réside d'une part dans le choix de l'agent promoteur d'absorption (« enhancer ») et d'autre part dans le choix de l'agent gélifiant qui est soit du Carbopol 980® soit du Carbopol 1382®.

Le passage percutané du principe actif est évalué par mesure de :
- la quantité cumulée de principe actif en fonction du temps (cf. exemple I),
- le pourcentage cumulé de principe actif en fonction du temps,
- et le flux de diffusion de principe actif en fonction du temps.

Le pourcentage de principe actif cumulé est le pourcentage total de la diffusion du principe actif à travers la peau sur une période déterminée.
Le flux de diffusion du principe actif s'exprime en µg / cm² / h : il permet de déterminer la cinétique de diffusion du principe actif dans le temps.

La méthode d'évaluation du passage du principe actif utilisée permet également de localiser la distribution de l'acétate de nomégestrol dans les différentes structures cutanées après diffusion.

Le tableau 9 ci-dessous représente le pourcentage cumulé d'acétate de nomegestrol en fonction du temps, ainsi que (cf. les 3 dernières lignes) les lieux de distribution du principe actif dans les structures cutanées, c'est-à-dire la teneur en acétate de nomegestrol dans les différentes couches (épiderme + derme) de la peau.

**Tableau 9 :**

| | **% cumulé en µg** | | | |
|---|---|---|---|---|
| | ***GELS*** | | | |
| | **G37-113** | **G32-104** | **G36-276** | **G36-264** |
| | 8 % PG 3 % Trans 0,6 % C980 | 8%PG 3 % Trans 0,5 % C1382 | 8 % PG 3 % Vit E 0,5 % C980 | 8 % PG 3 % Solkétal 0,5 % C980 |
| Temps en h | | | | |
| 0 | 0 | 0 | 0 | 0 |
| 2 | 0,042 | 0,058 | 0,105 | 0,183 |
| 4 | 0,088 | 0,135 | 0,193 | 0,371 |
| 6 | 0,137 | 0,227 | 0,275 | 0,554 |
| 8 | 0,19 | 0,329 | 0,347 | 0,732 |
| 10 | 0,239 | 0,402 | 0,405 | 0,894 |
| 24 | 0,575 | 1,117 | 0,667 | 1,926 |
| | | | | |
| Epiderme | 10,05 | 14,82 | 8,15 | 16,82 |
| Derme | 4,33 | 6,97 | 4,68 | 4,84 |
| Lavage | 67.63 | 61,15 | 72,69 | 60.35 |

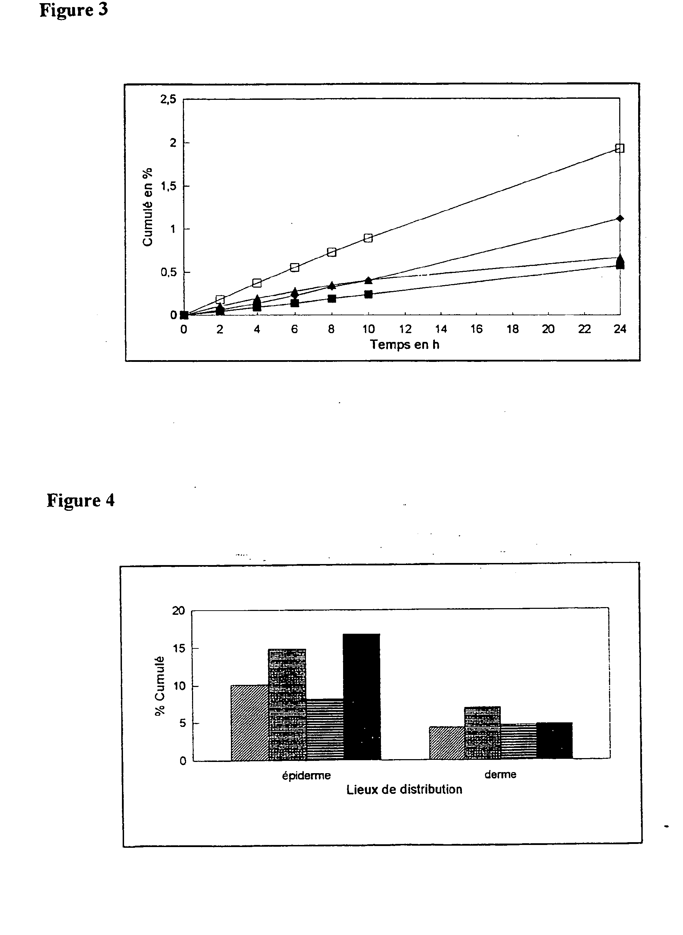

Le tableau 9 est illustré par les figures 3 et 4.
La figure 3 représente l'influence de l'enhancer (agent promoteur) et du Carbopol® sur le passage percutané du gel systémique d'acétate de nomégestrol.
Les agents promoteurs suivants sont comparés : Transcutol® (Tr), Solkétal (Sol) et Vitamine E TPGS (Vit E).

Les symboles ■, ▲ , ◆ et □ de la figure 3 représentent :

| | |
|---|---|
| ■ G37-113 (3 % Tr) | ◆ G32-104 (3 % Tr) - Réf. |
| ▲ G36-276 (3 % Vit E) | □ G36-264 (3 % Sol) |

La figure 4 représente la répartition de l'acétate de nomégestrol au niveau des structures cutanées.
Les symboles 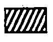 , 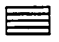 , 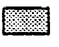 et 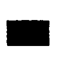 de la figure 4 représentent :

| | |
|---|---|
| 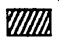 G37-113 (3 % Tr) | 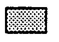 G32-104 (3 % Tr) - Réf. |
| 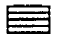 G36-276 (3 % Vit E) | 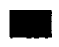 G36-264 (3 % Sol) |

A partir des valeurs de pourcentage cumulé de principe actif, on peut en déduire celles de quantité cumulée et de flux de diffusion.

La figure 5 représente le flux de diffusion d'acétate de nomegestrol en fonction du temps.
Les symboles ■, ▲, ◆ et □ de la figure 5 ont la même signification que ceux de la figure 3.
La cinétique de diffusion de l'acétate de nomegestrol avec les compositions sous forme de gels est de type patch, à diffusion constante.

### Conclusion:

Le Solkétal améliore le passage percutané de l'acétate de nomégestrol de façon plus importante que la Vitamine E et que le Transcutol® si l'on compare les résultats obtenus avec ceux du gel G32-104 de référence.
Ainsi, la meilleure diffusion est obtenue lorsque l'on utilise le Solkétal plutôt que la Vitamine E TPGS, alors que la solubilité du principe actif est meilleure pour cette dernière (cf. tableaux 5 et 6).

La même remarque peut être faite lorsque l'on est en système hydroalcoolique avec le mélange propylèneglycol / Transcutol® : si l'on considère les quatre combinaisons possibles 8:0 - 8:3 - 3:8 et 0:8, la diffusion obtenue est la meilleure pour le mélange 8:3. Or, c'est pour celui-ci que la solubilité est la moins bonne (cf. tableau 7).

Une certaine affinité du principe actif pour le solvant est nécessaire pour sa totale solubilisation. Cependant, elle ne doit pas être trop importante afin que le coefficient de partage entre le véhicule et la peau s'oriente en faveur de la diffusion à travers la peau.
Les essais de diffusion en flux statique sur principe actif radiomarqué ont été réalisés pour les gels contenant les deux types de promoteurs d'absorption retenus, contre deux gels de référence : le gel G32-104 (grade de carbopol différent que G37-113 et les 2 autres), pour lequel la meilleure diffusion a été obtenue et le gel G37-113, de même composition que les deux gels testés (même grade de Carbopol®) contenant du Transcutol®.
Si l'on considère l'action des promoteurs d'absorption retenus sur le passage percutané de l'acétate de nomégestrol marqué, on observe une nette augmentation de la diffusion en présence de Solkétal par rapport au gel de référence G32-104 renfermant du Transcutol®.

La vitamine E TPGS, utilisée dans les mêmes conditions, n'améliore pas le passage par rapport au gel G32-104. En revanche, si l'on considère le gel G37-113, la diffusion obtenue est légèrement améliorée avec la vitamine E, et très nettement augmentée avec le Solkétal.

Lorsque l'on observe la répartition quantitative du principe actif (cf figure 4) au niveau des structures cutanées, on retrouve pour le principe actif du gel G36-264 et du gel de référence G32-104, des concentrations similaires au niveau de l'épiderme et du derme. Celle-ci est moins importante au niveau de l'épiderme pour les gels G37-113 et G36-276.

Les essais ci-dessus confirment également qu'il existe une différence de diffusion de principe actif en fonction du grade de carbomère utilisé dans la formulation (les formulations des gels G37-113 et G32-104 sont quantitativement et qualitativement identiques à cette exception près). Or, il semble que la diffusion soit meilleure en présence de Carbopol 1382 ®, de même que la localisation au niveau des structures cutanées.
Si l'on considère les résultats obtenus en termes d'adhésion, lors des contrôles réalisés sur ces gels G36-264 et G36-276, on note que le caractère adhésif du gel contenant le Solkétal est légèrement supérieur à celui du gel contenant la vitamine E. Or, ces deux gels renferment les mêmes proportions des mêmes excipients, à l'exception de la nature du promoteur.

### Conclusion

Une composition hormonale topique à effet systémique actuellement préférée selon la présente invention est une composition sous forme de gel contenant :
- 0,4 % d'acétate de nomégestrol
- 8 % de propylèneglycol
- 3 % de Solkétal
- 0,5 % de Carbopol 980 ou 1382®
- 45 % d'éthanol 95°
- 0,05 % d'EDTA, 0,4 % de TEA et qsp 100% d'eau déminéralisée.

### 2 / Etude des formulations sous forme de solutions filmogènes ou filmantes

Les 5 solutions filmogènes étudiées sont dénommées « G36-259, G36-261, G36-263, G36-266 et G36-277 » et leurs formulations sont présentées dans le tableau 10 ci-dessous :

**Tableau 10**

| *FORMES* | *SOLUTIONS FILMOGENES* | | | | |
|---|---|---|---|---|---|
| Références | G36-259 | G36-261 | G36-263 | G36-266 | G36-277 |
| Formulations en % | | | | | |
| Nomégestrol Acetate | 0,4 | 0,4 | 0,4 | 0,4 | 0,4 |
| Propylèneglycol | 8 | 8 | 8 | 8 | 8 |
| Transcutol® | / | / | / | / | / |
| Solkétal | / | / | / | 3 | / |
| Vit E TPGS | / | / | / | / | 3 |
| HPMC 60SH4000 | / | / | / | / | / |
| Carbopol 1382® | / | / | / | / | / |
| Carbopol 980® | / | / | / | / | / |
| EDTA | 0,05 | 0,05 | 0,05 | 0,05 | 0,05 |
| TEA | / | 0,8 | 0,3 | 0,05 | 0,05 |
| Kollidon 90® | 5 | / | / | 5 | 5 |
| Aqoat AS-LF® | / | 10 | / | / | / |
| Eudragit L30D55® | / | / | 10 | / | / |
| Phtalate de diéthyle | / | 3 | 2 | / | / |
| Ethanol 95 | 43,35 | 40 | 40 | 43,25 | 43,25 |
| Eau déminéralisée | 43,2 | 37,75 | 39,25 | 40,25 | 40,25 |
| pH solution à 1 % | 6,25 | 6.16 | 6.24 | 6,83 | 6,34 |
| Viscosité mPa.s | | | | | |
| Titre AcN(%) | 0.41 | 0.42 | 0,43 | 0,40 | 0,40 |

La principale différence dans la composition de ces solutions filmogènes réside dans le choix de l'agent filmogène ainsi que dans le choix de l'ajout ou non, d'un promoteur d'absorption ou d'un agent plastifiant.

Les essais ont été réalisés pour les solutions filmogènes en comparaison avec le gel G32-104 comme référence.
Le tableau 11 ci-dessous représente le pourcentage cumulé d'acétate de nomégestrol en fonction du temps et la répartition de l'acétate de nomegestrol au niveau des structures cutanées.

**Tableau 11**

| | **Cumulé en %** | | | | | |
|---|---|---|---|---|---|---|
| | **GELS** | **SOLUTIONS FILMOGENES** | | | | |
| | **G32-104** | **G36-261** | **G36-263** | **G36-259** | **G36-266** | **G36-277** |
| | 8% PG | 8% PG | 8 % PG | 8% PG | 8% PG | 8% PG |
| Temps en h | 3% Trans | 10% Aqoat | 10% Eudr | 5% Koll | 3% Solk | 3% TPGS |
| | 0,5%C1382 | | | | 5% Koll | 5% Koll |
| 0 | 0 | 0 | 0 | 0 | 0 | 0 |
| 2 | 0,062 | 0,088 | 0,092 | 0,077 | 0,067 | 0,064 |
| 4 | 0,123 | 0,153 | 0,152 | 0,14 | 0,116 | 0,112 |
| 6 | 0,185 | 0,206 | 0,197 | 0,203 | 0,165 | 0,153 |
| 8 | 0,252 | 0,251 | 0,239 | 0,269 | 0,211 | 0,193 |
| 10 | 0,342 | 0,3 | 0,289 | 0,349 | 0,269 | 0,242 |
| 24 | 0,799 | 0,487 | 0,515 | 0,699 | 0,539 | 0,474 |
| | | | | | | |
| Epiderme | 8,24 | 5,69 | 2,78 | 9,14 | 6,41 | 4,43 |
| Derme | 5,56 | 1,93 | 2,26 | 4,58 | 3,2 | 4,76 |
| Lavage | 72,18 | 97,96 | 98,85 | 94,33 | 91,37 | 95,24 |

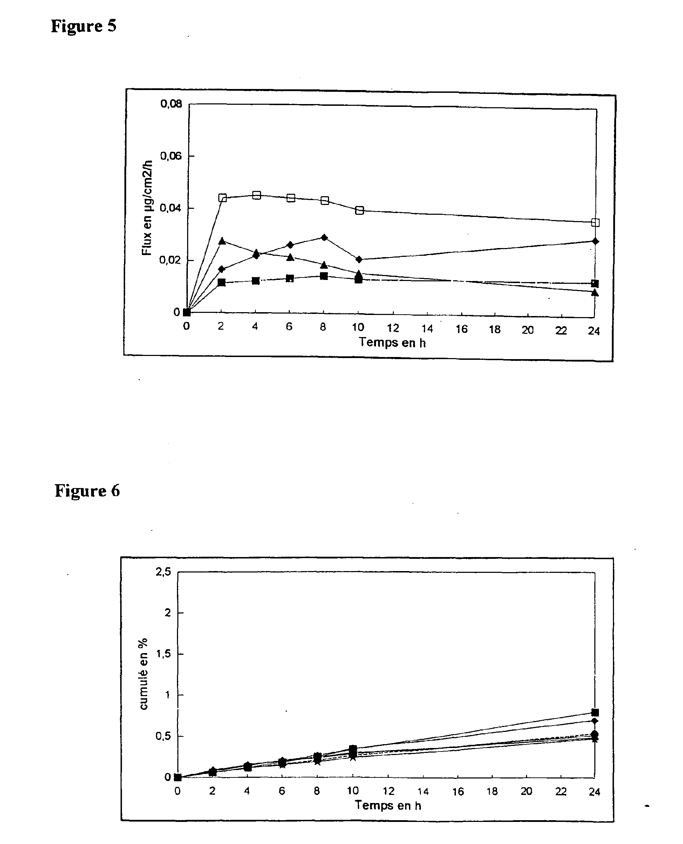

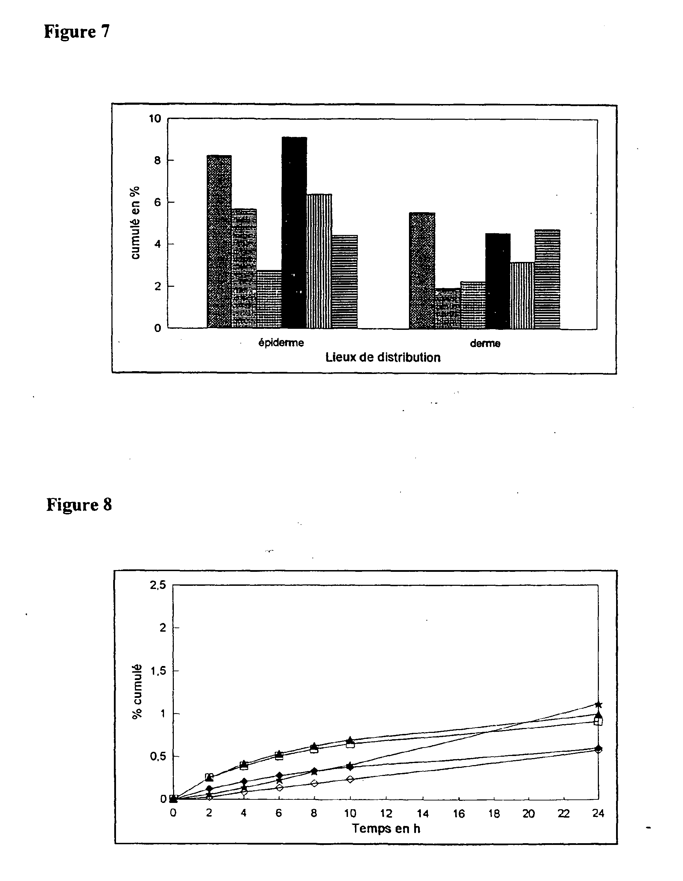

Le tableau 11 est illustré par les figures 6 et 7.

La figure 6 représente l'influence de l'agent promoteur d'absorption et de l'agent filmogène sur le passage percutané du film systémique d'acétate de nomégestrol.
Les symboles ■, ◆, ◇, ●, ▲ et ★ de la figure 6 représentent :

| | |
|---|---|
| ■ G32-104 (3%Tr) -Réf.- | ◇ G36-261 (10%Aqoat) |
| ◆ G36-259 (5%Kol) | ● G36-266 (3% Solk/5%Kol) |
| ▲ G36-263 (10%Eudrag) | |
| ★ G36-277 (3%TPGS/5%Kol) | |

Les quantités de principe actif ayant diffusé à partir de ces formes sont toutes inférieures aux quantités obtenues par application du gel G32-104 non filmogène, quel que soit le polymère considéré.
On constate que la solution ne contenant que du Kollidon® est celle qui donne la diffusion la plus proche de celle du gel de référence. Les deux autres polymères conduisent à des diffusions proches entre elles.
Les solutions associant le Kollidon® et un agent promoteur, comme le Solkétal ou la vitamine E TPGS, donnent des diffusions de principe actif inférieures par rapport à la solution G36-259 sans promoteur.
La figure 7 représente la distribution de l'acétate de nomegestrol au niveau des structures cutanées.
Les symboles 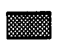 , 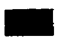 , 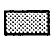 , 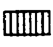 , 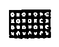 et 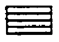 de la figure 7 représentent :

| | |
|---|---|
| 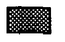 G32-104 (3%Tr) -Réf.- | 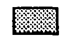 G36-261 (10%Aqoat) |
| 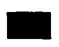 G36-259 (5%Kol) | 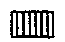 G36-266 (3% Solk/5%Kol) |
| 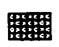 G36-263 (10%Eudrag) | |
| 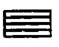 G36-277 (3%TPGS/5%Kol) | |

On constate que la meilleure répartition est pour le Kollidon®. Elle est équivalente à celle constatée pour le gel de référence. Les résultats obtenus pour les solutions d'Aqoat® et d'Eudragit® restent bas.

Les résultats de diffusion obtenus avec les solutions filmantes sont légèrement meilleurs que ceux obtenus avec le gel à 0,11 % d'acétate de nomegestrol (cf. tableau 1, exemple I). Cependant, il faut noter qu'en ce qui concerne l'Eudragit® et l'Aqoat®, les solutions réalisées ne contiennent que du propylèneglycol, sans autre agent promoteur, contrairement au gel de référence.

### Conclusion :

Une composition hormonale topique à effet systémique selon la présente invention sera par exemple une composition sous forme de solution filmogène contenant :
- 0,4 % d'acétate de nomégestrol
- 8 % de propylèneglycol
- 5 % de Kollidon 90®
- 43,35 % d'éthanol 95°
- 0,05 % d'EDTA et qsp 100% d'eau déminéralisée.

### 3 / Etude des formulations sous forme de gels filmogènes ou films gélifiés

Les trois gels filmogènes étudiés sont dénommés « G36-260, G36-262 et G36-267 » et leurs formulations sont présentées dans le tableau 12 ci-dessous.

**Tableau 12**

| *FORMES* | *GELS filmogènes* | | |
|---|---|---|---|
| Références | G36-260 | G36-262 | G36-267 |
| Formulations en % | | | |
| Nomégestrol Acetate | 0,4 | 0,4 | 0,4 |
| Propylèneglycol | 8 | 8 | 8 |
| Transcutol® | / | / | / |
| Solkétal | / | / | / |
| Vit E TPGS | / | / | / ' |
| HPMC 60SH4000 | / | / | 1 |
| Carbopol 1382® | / | / | / |
| Carbopol 980® | 0,5 | 0,75 | / |
| EDTA | 0,05 | 0,05 | 0,05 |
| TEA | 0,1 | 0,9 | 0,4 |
| Kollidon 90® | 5 | / | / |
| Aqoat AS-LF® | / | 10 | / |
| Eudragit L30D55® | / | / | 10 |
| Phtalate de diéthyle | / | 3 | 2 |
| Ethanol 95 | 43 | 40 | 40 |
| Eau déminéralisée | 42,95 | 36,9 | 38,15 |
| pH solution à 1 % | 6,36 | 6,2 | 6,17 |
| Viscosité mPa.s | 1750 | 1050 | 1150 |
| Titre AcN(%) | 0,41 | 0,405 | 0,40 |

La principale différence dans la composition de ces gels filmogènes réside dans le choix de l'agent gélifiant et de l'agent filmogène.
Ces essais ont été réalisés pour les gels filmogènes en comparaison avec les gels G32-104 et G37-113 comme référence.

Le tableau 13 ci-dessous représente le pourcentage cumulé d'acétate de nomégestrol en fonction du temps et la répartition de l'acétate de nomegestrol au niveau des structures cutanées.

| | **% Cumulé** | | | | |
|---|---|---|---|---|---|
| | **GEL** | | **GEL FILMOGENE** | | |
| | **G32-104** | **G37-113** | **G36-260** | **G36-262** | **G36-267** |
| | 8% PG | 8% PG | 8% PG | 8%PG | 8% PG |
| Temps en h | 3% Trans 0,5%C1382 | 3% Trans 0,6% C980 | 5% Kollidon 0,5% C980 | 10% aqoat 0,75% C980 | 10% Eudr 1% HPMC |
| 0 | 0,000 | 0,000 | 0,000 | 0,000 | 0,000 |
| 2 | 0,058 | 0,024 | 0,121 | 0,251 | 0,251 |
| 4 | 0,135 | 0,088 | 0,206 | 0,414 | 0,392 |
| 6 | 0,227 | 0,137 | 0,278 | 0,533 | 0,506 |
| 8 | 0,329 | 0,190 | 0,334 | 0,626 | 0,590 |
| 10 | 0,402 | 0,239 | 0,379 | 0,694 | 0,650 |
| 24 | 1,117 | 0,575 | 0,598 | 0,994 | 0,913 |
| Epiderme | 14,82 | 10,05 | 11,42 | 16,36 | 11,21 |
| Derme | 6,97 | 4,33 | 4,81 | 1,53 | 1,39 |
| Lavage | 61,15 | 67,63 | 65,83 | 72,35 | 90,42 |

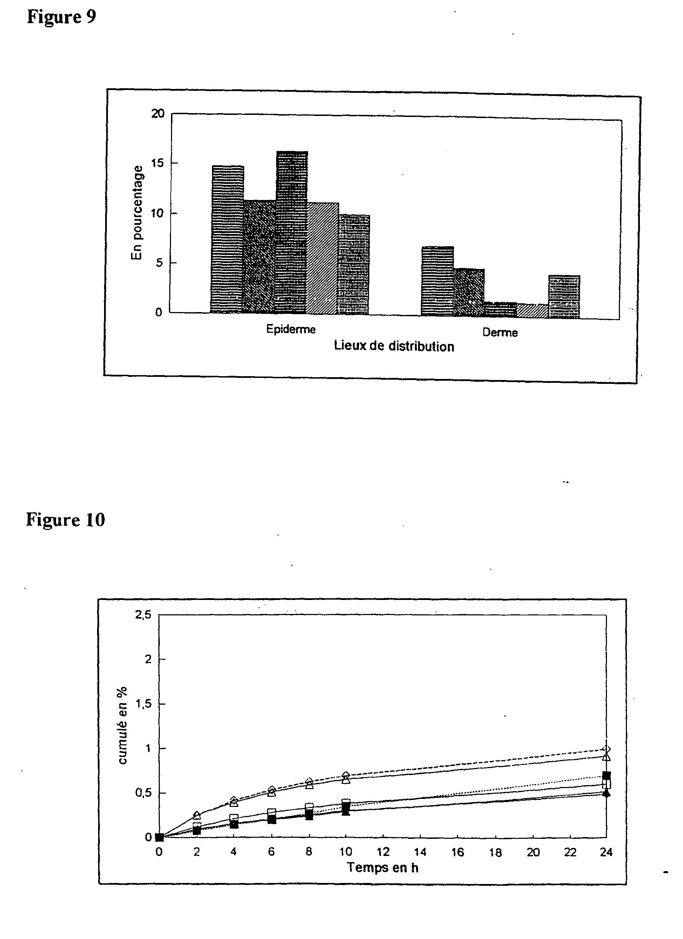

Le tableau 13 est illustré par les figures 8 et 9.
La figure 8 représente l'influence de l'agent filmogène sur le passage percutané du gel filmogène systémique d'acétate de nomégestrol.

Les symboles ★, □, ◆, ◇ et **▲** de la figure 8 représentent :

| | | |
|---|---|---|
| ★ G32-104 (C1382) | ◆ G36-260 (Kol/C980) | ▲ G36-262 (aq/C980) |
| □ G36-267 (Eud/HPMC) | ◇ G37-113 (C980) | |

Si l'on considère l'ensemble des polymères, la diffusion de l'acétate de nomégestrol à partir des gels filmogènes d'Aqoat® et d'Eudragit® est meilleure que pour le gel G32-104 « référence » jusqu'à 10 heures. Au-delà, la tendance s'inverse légèrement. Si l'on considère le gel 113 non filmogène, contenant un carbopol® différent du gel G32-104, les résultats obtenus pour tous les gels filmogènes sont meilleurs, quel que soit le polymère considéré.
On note que la diffusion du principe actif est similaire pour l'Aqoat® et l'Eudragit®.
En revanche, elle est très nettement plus basse pour le Kollidon®.

La figure 9 représente la localisation du principe actif au niveau des structures cutanées.

Les symboles 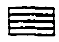 , 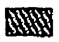 , 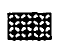 , 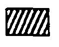 , 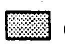 de la figure 9 représentent :

| | | |
|---|---|---|
| 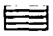 G32-104 (C1382) | 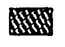 G36-260 (Kol/C980) | 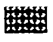 G36-262 (aq/C980) |
| 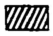 G36-267 (Eud/HPMC) | 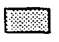 G37-113 (C980) | |

On observe que la localisation est variable d'un polymère à l'autre : par rapport au gel G32-104 de référence, la distribution au niveau de l'épiderme est semblable pour l'Aqoat®, et plus basse pour le Kollidon® et l'Eudragit®. La distribution au niveau du derme est plus basse pour l'Aqoat® et l'Eudragit®, et plus élevée pour le Kollidon®.

### Conclusion :

Des exemples de compositions hormonales topiques à effet systémique selon l'invention seront par exemple des compositions sous forme de gel filmogène contenant :
- 0,4 % d'acétate de nomégestrol
- 8 % de propylèneglycol
- 0,75 % de Carbopol 980®
- 10 % d'Aqoat AS-LF®
- 40 % d'éthanol 95°
- 3 % de phtalate de diéthyle, 0,05 % d'EDTA, 0,9 % de TEA et qsp 100% d'eau déminéralisée,
ou, des compositions sous forme de gel filmogène contenant :
- 0,4 % d'acétate de nomégestrol
- 8 % de propylèneglycol
- 1 % de HPMC 60 SH 4000
- 10 % d'Eudragit L 30 D 55®
- 40 % d'éthanol 95°
- 2 % de phtalate de diéthyle, 0,05 % d'EDTA, 0,4 % de TEA et qsp 100% d'eau déminéralisée.

### 4 / Comparaison entre des solutions filmogènes et gels filmogènes

Le tableau 14 ci-dessous représente le pourcentage cumulé d'acétate de nomégestrol en fonction du temps, et la répartition de l'acétate de nomegestrol au niveau des structures cutanées.

| | **% cumulé** | | | | | |
|---|---|---|---|---|---|---|
| | **SOLUTIONS FILMOGENES** | | | **GELS FILMOGENES** | | |
| | **G36-259** | **G36-261** | **G36-263** | **G36-260** | **G36-262** | **G36-267** |
| | 8% PG | 8% PG | 8 % PG | 8 % PG | 8% PG | 8% PG |
| Temps en h | 5 % Koll | 10% Aqoat | 10% Eudr | 5% Koll 0,5%C980 | 10%Aqoat 0,75%C980 | 10%Eudr 1%HPMC |
| 0 | 0 | 0 | 0 | 0 | 0 | 0 |
| 2 | 0,077 | 0,088 | 0,092 | 0,121 | 0,251 | 0,251 |
| 4 | 0,14 | 0,153 | 0,152 | 0,206 | 0,414 | 0,392 |
| 6 | 0,203 | 0,206 | 0,197 | 0,276 | 0,533 | 0,506 |
| 8 | 0,269 | 0,251 | 0,239 | 0,334 | 0,626 | 0,59 |
| 10 | 0,349 | 0,3 | 0,289 | 0,379 | 0,694 | 0,65 |
| 24 | 0,699 | 0,487 | 0,515 | 0,598 | 0,994 | 0,913 |
| Epiderme | 9,14 | 5,69 | 2,78 | 11,42 | 16,36 | 11,21 |
| Derme | 4,58 | 1,93 | 2,26 | 4,81 | 1,53 | 1,39 |
| Lavage | 94,33 | 97,96 | 98,85 | 65,83 | 72,35 | 90,42 |

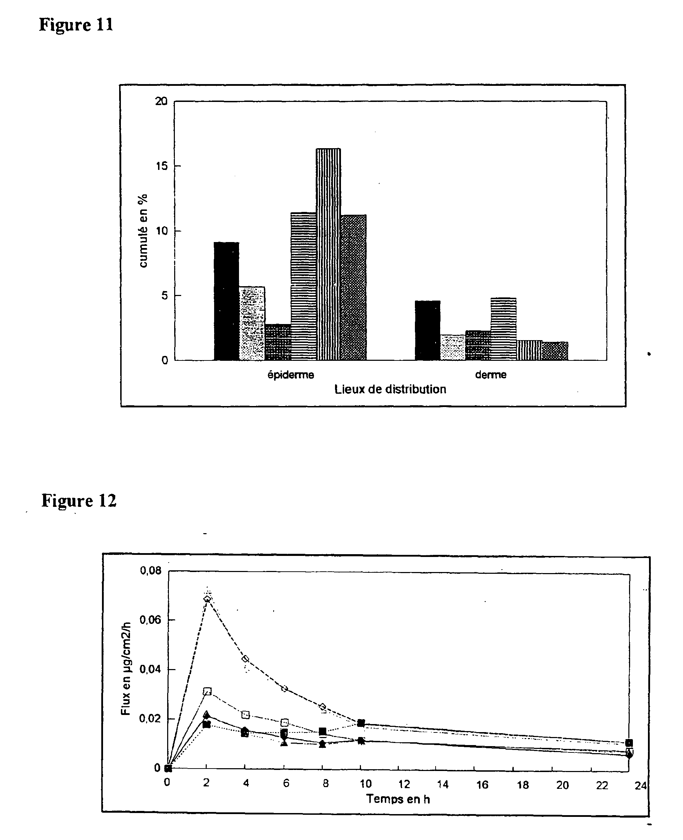

Le tableau 14 est illustré par les figures 10 et 11.
La figure 10 permet la comparaison des solutions filmogènes et des gels filmogènes d'acétate de nomégestrol à effets systémiques.

Les symboles ■, □, ◆, ◇, ▲ et △ de la figure 10 représentent :

| | | |
|---|---|---|
| Solutions filmogènes | | |
| ■ G36-259 (5% Kollidon) | ◆ G36-261 (10 % Aqoat) | ▲ G36-263 (10 % Eudrag) |
| Gels filmogènes | | |
| □ G36-260 (5% Kol/C980) | ◇ G36-262 (10%Aqoat/C980) | △ G36-267 (10 % Eudr/HPMC) |

La figure 11 représente la répartition du principe actif au niveau des structures cutanées.
Les symboles 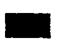 , 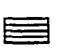 , 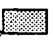 (colonne 2, fig. 11), 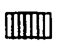 , 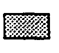 (colonne 3, fig. 11) et 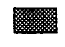 (dernière colonne) de la figure 11 représentent :

| | | |
|---|---|---|
| 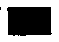 G36-259 (5% Kollidon) | 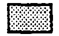 G36-261 (10 % Aqoat) | 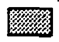 G36-263 (10 % Eudrag) |
| 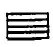 G36-260 (5% Kol/C980) | 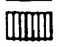 G36-262 (10%Aqoat/C980) | 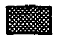 G36-267 (10 % Eudr/HPMC) |

La figure 12 compare les flux de diffusion d'acétate de nomegestrol avec des compositions sous forme de gel filmogène et avec des compositions sous forme de solution filmogène.

Les symboles de la figure 12 ont la même signification que ceux de la figure 10.

Contrairement à la figure 5 (flux de diffusion avec des compositions sous forme de gel), la cinétique de diffusion n'est pas une cinétique de diffusion constante, mais présente très rapidement (au bout de 2 heures) une diffusion maximale qui ensuite décroît assez rapidement. Ceci est particulièrement vrai pour les gels filmogènes G36-262 et G36-267. Ainsi, on distinguera deux types de flux : des flux à diffusion plus ou moins constante, et d'autres présentant très rapidement un pic de diffusion maximum.

Ainsi, les gels filmogènes sont mieux adaptés qu'une solution filmogène pour l'optimisation du partage percutané de l'acétate de nomégestrol. Plus particulièrement, la seule présence d'un agent filmogène de nature cellulosique (Aqoat®, G36-262) ou acrylique (Eudragit®, G36-267) dans un gel filmogène permet d'obtenir une bonne diffusion du principe actif. La pellicule formée, dans les deux cas, est à la fois plus résistante, cohésive, et semble permettre la libération du principe actif.

Dès lors, on peut envisager d'associer en outre aux compositions hormonales topiques à effet systémique sous forme de gel filmogène, 3 % de Solkétal afin d'obtenir une synergie d'action et améliorer encore la diffusion de l'acétate de nomégestrol.

### 4 / Conclusion

Les solutions filmogènes ou solutions filmantes ne permettent en général que des diffusions de principe actif inférieures à celles obtenues pour le gel de référence (G32-104).
En revanche, les gels filmogènes d'Aqoat® (G36-262) et d'Eudragit® (G36-267) permettent d'obtenir des diffusions intéressantes de principe actif, si l'on considère les formulations réalisées ne renfermant aucun agent promoteur d'absorption.

Le Solkétal est un agent promoteur d'absorption qui semble actif sur la diffusion de l'acétate de nomégestrol à travers la peau ; en effet, en système hydroalcoolique et en association avec le propylèneglycol dans les proportions (3:8), il permet d'améliorer sensiblement sa solubilité dans le véhicule et son passage percutané à travers la peau.
Ainsi, un exemple particulièrement approprié d'une composition hormonale topique à effet systémique selon l'invention est une composition qui se trouve sous forme de gel ou de gel filmogène et qui renferme dans un mélange hydroalcoolique 8 % de propylèneglycol et-3 % d'isopropylidène glycérol.

### • EXEMPLE IV

### Essais cliniques préliminaires

Dans ces exemples, les essais cliniques ont été effectués sur les femmes avec le gel contenant 0,11 % d'acétate de nomegestrol, dont la formulation est donnée dans le tableau 1 de l'exemple I.

### 1/ Exemple clinique n° 1

Vingt-quatre femmes, volontaires, en bonne santé et en période d'activité ovarienne, âgées en moyenne de 23,5 ans ont été traitées pendant 15 jours consécutifs par 4 mg d'acétate de nomégestrol dans un gel appliqué chaque jour sur les deux seins.

Des prélèvements sanguins répétés ont été faits dans les heures suivant la première et la dernière administration, ainsi qu'à 9 reprises (6 fois avant l'application du gel et 3 fois 3 heures après), entre le 2e et le 14e jour de traitement. L'acétate de nomégestrol a été dosé dans le plasma de ces échantillons par chromatographie liquide couplée à la spectrométrie de masse.

Dès le premier jour de traitement, l'acétate de nomégestrol a pu être dosé chez tous les sujets. La concentration maximale a été évaluée à 0,25 ± 0,027 ng/ml et l'aire sous la courbe de 0 à 48 heures, de 6,08 ± 0,775 ng/ml par h, les taux moyens s'établissant en plateau entre 0,10 et 0,17 ng/ml.

Après la dernière administration, la concentration maximale était de 0,65 ± 0,073 ng/ml et l'aire sous la courbe de 0 à 48 heures de 18,43 ± 2,091 ng/ml par h, l'acétate de nomégestrol étant encore détecté dans le plasma 72 heures après la dernière application (taux à 0,19 ± 0,027 ng/ml).

L'état d'équilibre est obtenu après le 3e jour de traitement. On observe alors des taux moyens restant en plateau et oscillant peu, entre 0,42 et 0,65 ng/ml.

### 2/ Exemple clinique n° 2

Six femmes ménopausées, âgées de 56 à 66 ans et sans traitement hormonal substitutif depuis 2 mois ont été suivies pendant 2 cycles consécutifs de 25 jours, séparés par une fenêtre thérapeutique de 6 jours.

Durant chaque jour, elles ont reçu un comprimé d'estradiol oral par jour et, pendant les 15 jours du second cycle, 4 mg d'acétate de nomégestrol appliqués sous forme d'un gel sur la peau abdominale. A la fin de chaque cycle, l'acétate de nomégestrol a été dosé dans le plasma, la survenue des hémorragies génitales a été notée et une biopsie de l'endomètre a été réalisée.

Contrairement à ce que l'on a observé au cours du premier cycle (estradiol seul), lors du second cycle, l'administration du gel d'acétate de nomégestrol a permis de constater que le progestatif était détectable dans le plasma à des taux compris entre 0,39 et 0,76 ng/ml (0,62 ng/ml en moyenne) et que ces taux ont été suffisants pour entraîner une transformation sécrétoire de l'endomètre et obtenir une hémorragie génitale, en moyenne 5 jours après la fin du second cycle.

### 3/ Exemple clinique n° 3

Cent treize femmes non ménopausées et souffrant de douleurs mammaires depuis plus de 3 mois et durant au moins 7 jours par cycle ont été traitées pendant une durée moyenne de 130 jours par 4 mg d'acétate de nomégestrol appliqués chaque jour, les 15 derniers jours du cycle menstruel, sous forme d'un gel sur les deux seins.

L'efficacité a été jugée après 3 mois et en fin de traitement grâce à une échelle visuelle analogique permettant de quantifier la douleur mammaire.

Cette évaluation a permis de constater que le gel d'acétate de nomégestrol diminuait de façon statistiquement significative l'intensité et la durée dans le cycle de la douleur mammaire, et ceci dès le 3e mois de traitement. Après 6 cycles de traitement, l'intensité avait diminué de 48 % et la durée de 41 %.

Au cours de cette étude, 55 femmes ont bénéficié d'un dosage de l'acétate de nomégestrol dans le sang, ce qui a permis d'observer des valeurs de 0,44 ± 0,30 (m ± sd) ng/ml.

## Revendications

1. Composition hormonale topique sous forme d'un gel à effet systémique, **caractérisée en ce qu'**elle contient:
- à titre de principe actif, le nomégestrol, ses esters et/ou ses éthers à la concentration de 0.05% à 1% en poids total de la composition,
- un véhicule permettant d'obtenir le passage systémique dudit principe actif constitué par un agent solubilisant formé d'un mélange d'au moins deux solvants choisis dans le groupe constitué par l'eau, les alcools, le propylène glycol, le polyéthylèneglycol, le polyéthylène 20-sorbitane monoléate, un glycéride en C₈/C₁₀ polyoxyéthylène glycosylé,
- un promoteur d'absorption
- un agent filmogène et/ou un agent gélifiant,
en association ou en mélange avec des excipients appropriés pour la réalisation d'une forme pharmaceutique filmogène et/ou gélifiée assurant des taux thérapeutiques efficaces.

2. Composition hormonale topique à effet systémique selon la revendication 1 ou 2, **caractérisée en ce que** le principe actif est l'acétate de nomégestrol.

3. Composition hormonale topique à effet systémique selon l'une des revendications précédentes, **caractérisée en ce que** la quantité de nomégestrol et/ou d'un de ses esters ou éthers est de 0,1 à 0,8% en poids de la composition totale.

4. Composition hormonale topique à effet systémique selon l'une des revendications 1 à 4, **caractérisé en ce que** l'agent solubilisant est un mélange binaire formé d'éthanol à 95 ° et d'eau dans lequel le pourcentage d'éthanol à 95 ° varie de 30 à 50 %.

5. Composition hormonale topique à effet systémique selon l'une des revendications 1 à 4, **caractérisée en ce que** l'agent solubilisant est un mélange ternaire éthanol à 95° / eau / propylèneglycol, dans lequel le pourcentage d'éthanol à 95° varie de 30 à 50 %, celui d'eau de 30 à 60 % et celui de propylèneglycol de 2 à 20 %.

6. Composition hormonale topique à effet systémique selon l'une des revendications 1 à 4, **caractérisée en ce que** l'agent solubilisant est un mélange quaternaire éthanol à 95° / eau / Labrasol® / propylèneglycol, dans lequel le pourcentage d'éthanol à 95° varie de 30 à 50 %, celui d'eau de 30 à 60 %, celui de Labrasol® de 3 à 7 % et celui de propylèneglycol de 2 à 20%.

7. Composition hormonale topique à effet systémique selon l'une des revendications 2 à 7, **caractérisée en ce que** l'agent promoteur d'absorption est choisi dans le groupe constitué par l'isopropylidèneglycérol, l' α-tocophéryl polyéthylèneglycol 1 000 succinate.

8. Composition hormonale topique à effet systémique selon la revendication 8, **caractérisée en ce que** l'agent promoteur d'absorption est l'isopropylidèneglycérol.

9. Composition hormonale topique à effet systémique selon l'une des revendications 1 à 9, **caractérisée en ce que** l'agent gélifiant est choisi dans le groupe constitué par les dérivés cellulosiques et les dérivés acryliques.

10. Composition hormonale topique à effet systémique selon la revendication 10, **caractérisée en ce que** le dérivé cellulosique est l'hydroxypropylméthylcellulose

11. Composition hormonale topique à effet systémique selon la revendication 11, **caractérisée en ce que** le dérivé acrylique est un carbomère.

12. Composition hormonale topique à effet systémique selon l'une des revendications 1 à 12, **caractérisée en ce que** l'agent filmogène est choisi dans le groupe constitué par les dérivés cellulosiques, les dérivés méthacryliques et les dérivés de la polyvinylpyrrolidone.

13. Composition hormonale topique à effet systémique selon la revendication 13, **caractérisée en ce que** le dérivé cellulosique filmogène est l'acétate succinate d'hydroxypropylméthylcellulose.

14. Composition hormonale à effet systémique selon la revendication 13, **caractérisée en ce que** le dérivé méthacrylique est une dispersion aqueuse d'un copolymère anionique d'acide méthacrylique et d'acrylate d'éthyle.

15. Composition hormonale à effet systémique selon l'une quelconque des revendications 1 à 15, sous forme de gel ou de gel filmogène **caractérisée en ce qu'**elle renferme dans un mélange hydroalcoolique, 8 % de propylèneglycol et 3 % d'isopropylidène glycérol.

16. Utilisation d'une composition selon l'une des revendications 1 à 16 pour la réalisation d'un médicament à effet systémique par application topique, destiné à la correction des carences en progestérone chez la femme non ménopausée ou à une substitution hormonale chez la femme ménopausée.

## Claims

1. Topical hormonal composition in the form of a gel, with systemic effect, **characterized in that** it contains :
- as active principale, nomegestrol and/or one of its ethers or esters at a concentration from 0.05 % to 1 % by weight of the total composition,
- a vehicle permitting the systemic passage of the said active principle, constituted by a solubilizing agent formed by a mixture of a least two solvents selected from the group comprising water, alcohols, propyleneglycol, polyethylene glycol, polyethylene 20-sorbitane monoleate, a C8/ C10 polyoxyethylene glycosyl glyceride or their mixtures,
- an absorption promoter,
- a film-forming agent and/or a gelling agent
combined with or mixed with suitable excipients for the production of a gelled and/or film-forming pharmaceutical form ensuring efficient therapeutical levels.

2. Topical hormonal composition with systemic effect according to claim 1, wherein the active principle is nomegestrol acetate..

3. Topical hormonal composition with systemic effect according to any of the preceding claims wherein the amount of nomegestrol or of one of its esters or ethers ranges from 0.1 to 0.4 % by weight of the total composition.

4. Topical hormonal composition with systemic effect according to any one of the claims 1 to 3, **characterized in that** the solubilizing agent is a binary mixture of 95° ethanol and water, in which the percentage of 95° ethanol ranges from 30 to 50 %.

5. Topical hormonal compositions with systemic effect according to any one of the claims 1 to 4, wherein the solubilizing agent is a ternary mixture of 95° ethanol / water / propyleneglycol, in which the percentage of 95° ethanol ranges from 30 to 50 %, that of water 30 to 60 % and that of propyleneglycol from 2 to 20 %.

6. Topical hormonal composition with systemic effect according according to any one of the claims 1 to 3, **characterized in that** the solubilizing agent is a quaternary mixture 95° ethanol / water / Labrasol® / propyleglycol, in which the percentage of 95° ethanol ranges from 30 to 50 % that of water 30 to 60 %, that of Labrasol® from 3 to 7 % and that of propyleneglycol from 2 to 20 %.

7. Topical hormonal composition with systemic effect according to any one of the claims 1 to 6, **characterized in that** the absorption promoter is selected from the group consisting of isopropyledeneglycerol and α-tocopheryl propylene glycol 1000 succinate.

8. Topical hormonal composition with systemic effect according to claim 7, **characterized in that** the absorption promoter is isopropyledeneglycerol

9. Topical hormonal composition with systemic effect according to any one of the claims 1 to 10, **characterized in that** the gelling agent is selected from the group consisting of cellulose derivatives and acrylic derivatives.

10. Topical hormonal composition with systemic effect according to claim 9, wherein that the cellulose derivative is hydroxypropylmethylcellulose.

11. Topical hormonal composition with systemic effect according to claim 7, **characterized in that** the acrylic derivative is a carbomer.

12. Topical hormonal composition with systemic effect according to any one of the claims 1 to 11, **characterized in that** the film-forming agent is selected from the group consisting of cellulose derivatives, methacrylic derivatives and polyvinylpyrrolidone derivatives.

13. Topical hormonal composition with systemic effect according to claim 12, **characterized in that** the film-forming cellulose derivative is hydroxypropylmethylcellulose acetate succinate.

14. Topical hormonal composition with systemic effect according to claim 12, **characterized in that** the methacrylic derivative is an aqueous dispersion of an anionic copolymer of methacrylic acid and ethyl acrylate.

15. Topical hormonal composition with systemic effect according to claims 1 to 14, **characterized in that** it is in the form of a gel or a film-forming gel and **in that** it contains, in an aqueous-alcoholic mixture, 8 % of propyleneglycol and 3 % of isopropylidene glycerol.

16. Use of a composition according to any of the claims 1 to 15, for the manufacturing of a medicament with systemic effect by topical application, for the correction of progesterone deficiency in pre-menopausal women and for hormone replacement in menopausal women.

## Patentansprüche

1. Topische hormonale Zusammensetzung in der Gestalt eines Gels, **dadurch gekennzeichnet dass** sie.
als der Wirkstoff, Nomegestrol, eine seine Estern und/oder seinen Ethern, mit der Konzentration von 0,5% bis 1% der Gesamtgewicht der Zusammensetzung.
einen Vehikel der den systematischen Durchang von den genannten Wirkstoffe erlaubt, das aus einen auflösende Mittel das von einen Mischung von mindenstens zwei Lösungsmittel aus der Gruppe von Wasser, Alkoholen, Propyleneglykol, Polyethyleneglykol, Polyethylene 20-sorbiban monooleate, einen Glycerid in C8-C10 polyoxyethylene glykosiliert, oder die Mischungen davon, gewählt ist, besteht.
- einen Absorption Beföi derer
- einen Filmbildendes Mittel und/oder einen Gelbildendes Mittel enthält
in Verbindung or in Mischung mit geeignete Verdunnungsmitteln, um die Herstellung einer Filmbildende und/oder Gelbildende pharmazeutische Bereitung die therapeutische Verhältnisse versichert, zerverwirklichen.

2. Topische hormonale Zusammensetzung nach Anspruch 1, **dadurch gekennzeichnet dass** der Wirkstoff Nomegestrol Acetat ist.

3. Topische hormonale Zusammensetzung die eine systematischen Wirkung besitzt, nach einer der vorstehenden Ansprüche, **dadurch gekennzeichnet dass** die Menge von nomegestrol, und/oder einen seiner Estern, oder Ethern, von 0,1 à 0,4 % der Gewichts der gesamte Zusammensetzung enthält.

4. Topische hormonale Zusammensetzung nach einer die Ansprüche 1 bis 3, **dadurch gekennzeichnet daß** das auflösende Mittel einen binären Mischung die mit 95 % Ethanol und Wasser besheht worin der Prozent von 95 % Ethanol, von 30 bis 50% sich verteilt.

5. Topische hormonale Zusammensetzung die eine systematische Wirkung besitzt, nach einer der Ansprüche 1 bis 4, **dadurch gekennzeichnet daß** das auflösendes Mittel, eine ternären Mischung von 95° Ethanol/ Wasser / Propyleneglycol ist, in der das Prozent von 95° Ethanol von 30 bis 50 %, das Prozent von Wasser von 30 bis 60 % und das von Propyleneglykol von 2 bis 20 % sich verteilt.

6. Topische hormonale Zusammensetzung nach einer der Ansprüche 1 bis 4 **dadurch gekennzeichnet daß** das auflösendes Mittel eine quaternäre Mischung von 95° Ethanol/Wasser/Labrasol® /Propyleneglycol ist, worin das Prozent von 95° Ethanol von 30 bis 50 %, das Prozent von Wasser von 30 bis 60%, das Prozent von Labrasol® von 3 bis 7% und das Prozent von Propyleneglycol von 2 bis 20% sich verteilt.

7. Topisch hormonale Zusammensetzung die eine systemische Wirkung besitzt, nach eine der Ansprüche 1 bis 6, **dadurch gekennzeichnet ist daß** der Absorptionsbeförderer aus der Gruppe von Isopropylidene glycerol und α-Tocopheryl polyéthylene glykol 1000 bernsteinsäure ester, gewählt ist.

8. Topische hormonale Zusammensetzung die eine systemische Wirkung besitzt, nach Ansprüche 7 **dadurch gekennzeichnet ist** dast der Absorptionsbeförderer Isopropylidene glycerol ist.

9. Topische hormonale Zusammensetzung die eine systemische Wirkung besitzt, nach einer der Ansprüche 1 bis 8, **dadurch gekennzeichnet ist, dass** das Gelbildendes Mittel aus der Gruppe von Zellstoffe Derivaten und Acrylsäure Derivaten gewählt ist.

10. Topische hormonale Zusammensetzung die systemischen Eigenschaften besitzt, nach Ansprüch 9 **dadurch gekennzeichnet ist daß** der Zellstoff Derivate Hydroxypropylcellulose ist.

11. Topische hormonale Zusammensetzung die systemischen Eigenschaften besitzt, **dadurch gekennzeichnet ist daß** die Acrylsäure ein Carbomer ist.

12. Topische hormonale Zusammensetzung die systemischen Eigenschaften besitzt, nach Ansprüche 1 bis 11, **dadurch gekennzeichnet ist daß** das Filmbildendes Mittel aus der Gruppe von Zellstoff Derivaten, Methacrylsaüre Derivaten und Polyvinylpyrrolidone Derivaten, gewählt ist.

13. Topische hormonale Zusammensetzung die systemischen Eigenschaften besitzt, **dadurch gekennzeichnet daß** die filmbildende Zellstoff Derivate Hydroxypropylmethylcellulose acetate bernsteinsaüre ester ist.

14. Topische hormonale Zusammensetzung die systemischen Eigenschaften Wirkung besitzt, nach Ansprüch 12, **dadurch gekennzeichnet daß** der Methacrylsäure Derivat ein wässrige Dispersion eines anionischen Derivates von Methacrylsäure und Ethyl Acrylat ist.

15. Topische hormonale Zusammensetzung die systemischen Eigenschaften besitzt, nach einer der Ansprüche 1 bis 14, **dadurch gekennzeichnet daß** diese in der Form eines Gels oder eines Filmbildende Gel ist, und dass sie einen Mischung von Wasser und Alkohol, 8 % Propylene glykol und 3% Isopropylidene glycerol enthält.

16. Verwendung einer Zusammensetzung nach einer der Ansprüche 1 bis 15 für die Herstellung eines Arzneimitels das systemische Eigenschaften besitzt bei topischen Anwendung, für die Verbesserung von Progesteron-Mangel bei pre-klimakteriung Frauen und für den Hormone Ersatz bei menoposal Frauen.
